# EUROPÄISCHE PATENTANMELDUNG

(11) **EP 3 117 786 A1**
(43) Veröffentlichungstag der Anmeldung: **18.01.2017**
(21) Anmeldenummer: 16172260.8
(22) Anmeldetag: 31.05.2016
(51) Int. Cl.: A61B 17/3205, A61B 17/3207

(54) **INSTRUMENT ZUM ENTFERNEN VON KÖRPERGEWEBE AUS EINEM FISTELGANG**

(30) Priorität: 13.07.2015 AT 4602015
(71) Anmelder: A.M.I. Agency for Medical Innovations GmbH, 6800 Feldkirch (AT)
(72) Erfinder: Hohlrieder, Martin, 6840 Götzis (AT)
(74) Vertreter: Fechner, Thomas

(57) **Zusammenfassung**

Instrument zum Entfernen von Körpergewebe aus einem Fistelgang (1) mit einem eine Schneide (12) aufweisenden Schneidwerkzeug (10) und einer Führungsstange (30) zum Durchführen durch den Fistelgang (1). Das Schneidwerkzeug (10) ist rohrförmig ausgebildet und auf die Führungsstange (30) aufschiebbar. Das Schneidwerkzeug (10) weist einen hinteren Führungsabschnitt (16) zur verschiebbaren Führung des Schneidwerkzeugs (10) auf der Führungsstange (30) und einen vorderen Schneidabschnitt (11) auf, an dem an einem vorderen Ende die Schneide (12) ausgebildet ist und dessen Innendurchmesser (15) zur Ausbildung eines Aufnahmehohlraums (14) für die Aufnahme von abgetrenntem Körpergewebe (5) größer als der Außendurchmesser (34) der Führungsstange (30) ist. (Fig. 8)

## Beschreibung

Die vorliegende Erfindung betrifft ein Instrument zum Entfernen von Körpergewebe aus einem Fistelgang mit einem eine Schneide aufweisenden Schneidwerkzeug.

Fisteln sind nicht natürlich vorbestehende, röhren- oder röhrennetzartige Verbindungen zwischen einem inneren Hohlorgan und anderen Organen oder der Körperoberfläche von Lebewesen. Der Körper kann einen oder mehrere Fistelgänge bilden, um den bei einer Entzündung entstehenden Eiter aus einem Abszess abzutransportieren.

Fisteln, beispielsweise im Bereich des Afters, sogenannte Analfisteln, sind für die Betroffenen sehr unangenehm. Der Fistelgang verbindet dabei das Rektum mit der Körperoberfläche im Bereich des Anus des Patienten. Analfisteln weisen oft eine epitheliale Auskleidung auf, welche eine Fistelwand des Fistelgangs bildet und eine Stärke von ca. 0,5 mm oder mehr aufweist. Die epitheliale Auskleidung erschwert das selbständige Zusammenwachsen und Ausheilen der Fistel. Die Länge des Fistelgangs von Analfisteln liegt häufig im Bereich von 2 cm bis 6 cm. Der Durchmesser des Fistelgangs beträgt in der Regel mehr als 2 mm.

Es sind verschiedene Therapien zur Behandlung von Fisteln, insbesondere von Analfisteln, bekannt. Bei Bedarf wird dabei zuerst der Eiter über eine Fadendrainage aus der Fistel abgeleitet. Hierzu wird ein Faden mittels einer stabförmigen Einführhilfe in den Fistelgang eingeführt und die beiden Enden des Fadens außerhalb des Körpers des Patienten verknotet. Nach Möglichkeit werden Fisteln dann unter Schonung des Schließmuskels ausgeschnitten oder gespalten, um ein Ausheilen der Fistel zu ermöglichen. Dies ist insbesondere bei nahe dem Schließmuskel befindlichen Fistelgängen problematisch, da eine radikale Entfernung die Kontinenz des Patienten gefährden kann.

Durch Einsetzen eines Fistelverschlusses von innen in den Fistelgang, z.B. aus einem resorbierbaren Implantat, kann das Ausmaß des operativ zu entfernenden Körpergewebes reduziert werden. Eine andere Fistelverschluss-Technik besteht in der thermischen Behandlung des Fistelgangs, z.B. mittels Laser, um den Fistelgang zu destruieren und zu okkludieren.

Eine weitere Therapieform besteht darin, mittels einer Kürette, d.h. einem stabförmigen Instrument mit einem verbreiterten vorderen Ende, an dem eine Schneide ausgebildet ist, die Fistelwand manuell abzutragen, um eine spätere Ausheilung der Fistel zu ermöglichen. Dazu ist es nötig, die außenliegenden Fistelabschnitte im Bereich der Öffnungen großräumig auszuschneiden, z.B. in Trichterform, um ein Ausheilen der Fistel von innen heraus zu ermöglichen. Zum Abtragen der Fistelwand ist eine Vielzahl von Küretten erforderlich, da diese schnell stumpf werden. Der Eingriff ist zeitintensiv und erfordert eine hohe Konzentration des ausführenden Personals, damit die Fistelwand vollständig abgetragen wird. Verbleiben Reste der Fistelwand im Fistelgang, können neue Entzündungen entstehen, wodurch die Fistel häufig nicht zufriedenstellend ausheilen kann.

Aufgabe der Erfindung ist es, ein vorteilhaftes Instrument der eingangs genannten Art bereitzustellen, welches eine zuverlässige und einfache Entfernung von Körpergewebe aus einem Fistelgang ermöglicht.

Erfindungsgemäß gelingt dies durch ein Instrument mit den Merkmalen des Anspruchs 1.

Das Instrument gemäß der Erfindung weist eine Führungsstange zum Durchführen durch den Fistelgang auf. Im Weiteren ist ein rohrförmig ausgebildetes Schneidwerkzeug auf die Führungsstange aufschiebbar. Das Schneidwerkzeug weist einen hinteren Führungsabschnitt zur verschiebbaren Führung des Schneidwerkzeugs auf der Führungsstange und einen vorderen Schneidabschnitt auf. An einem vorderen Ende des Schneidabschnitts ist die Schneide ausgebildet. Der Innendurchmesser des Schneidabschnitts ist zur Ausbildung eines Aufnahmehohlraums für die Aufnahme von abgetrenntem Körpergewebe größer als der Außendurchmesser der Führungsstange.

Die Führungsstange dient einerseits der sogenannten Darstellung des Fistelgangs, d.h., dass der Fistelgang vor der Durchführung des Schneidvorgangs entsprechend ausgerichtet ist. Der Außendurchmesser der Führungsstange ist dabei vorzugsweise so gewählt, dass der Fistelgang auf der Führungsstange aufgespannt ist, d.h. dass der Fistelgang durch die eingeführte Führungsstange leicht aufgeweitet ist, wobei die Fistelwand günstigerweise über die gesamte Längserstreckung des Fistelgangs an der Führungsstange anliegt. Andererseits dient die Führungsstange auch der Führung des Schneidwerkzeugs entlang der Längserstreckung der Führungsstange. Die Führungsstange könnte im Sinne der Erfindung auch als Stab oder Führungsstab bezeichnet werden. Vorzugsweise ist die Führungsstange gerade ausgebildet.

Durch die definierte Führung des Schneidwerkzeugs auf der Führungsstange in Richtung der Längsmittelachse der Führungsstange kann die Gefahr von unerwünschten Verletzungen des umliegenden Körpergewebes vermindert werden, da der Freiheitsgrad des Schneidwerkzeugs in Bezug auf die Führungsstange vorteilhafterweise auf eine axiale Richtung (in Richtung der Längsmittelachse der Führungsstange) und auf eine Verdrehung um die Längsmittelachse der Führungsstange eingeschränkt ist. Zudem kann eine Entfernung des Körpergewebes dadurch auf eine definiertere Art und Weise erfolgen.

Vorzugsweise liegt die Schneidkante der Schneide des Schneidwerkzeugs, in einer axialen Ansicht des Schneidwerkzeugs, auf einem Kreis, welcher beim auf die Führungsstange aufgeschobenen Schneidwerkzeug konzentrisch zur Längsmittelachse der Führungsstange liegt. Durch Verdrehung des Schneidwerkzeugs um die Längsmittelachse der Führungsstange und bei gleichzeitiger Vorwärtsbewegung in Richtung der Längsmittelachse kann die den Fistelgang umgebende Fistelwand als geometrisch definierter Hohlzylinder vom umliegenden Körpergewebe abgetrennt werden.

Die Schneide kann durchgehend kreisförmig ausgebildet sein, d.h., dass die Schneidkante der Schneide dann kreisrund ist. Es wäre beispielsweise aber auch denkbar und möglich, dass die Schneidkante einen Wellenschliff aufweist. Auch bei einem Wellenschliff liegt die Schneidkante der Schneide, in einer axialen Ansicht des Schneidwerkzeugs gesehen, vorzugsweise auf einem Kreis.

Das rohrförmig ausgebildete Schneidwerkzeug könnte auch als Schneidrohr bezeichnet werden. Schneidrohre zum Einsatz in medizinischen Anwendungen sind grundsätzlich bekannt, beispielsweise zur laparoskopischen Hysterektomie und zur Myomenukleation. Im Bereich der minimal-invasiven Chirurgie sind sogenannte Morcellatoren bekannt, die dem Zerkleinern von großen, aus dem Körperinneren zu entfernenden, Gewebeteilen oder Organen dienen. Dabei dreht sich ein von einem Schutzrohr umgebenes, motorisch angetriebenes Schneidrohr des Morcellators mit hoher Drehzahl. Die zerkleinerten Gewebeteile werden durch den Innenhohlraum des Schneidrohrs nach außen transportiert.

Der Führungsabschnitt weist bezogen auf die Richtung der Längsmittelachse des Schneidwerkzeugs günstigerweise eine Länge von zumindest 1 cm, bevorzugt von zumindest 3 cm, auf, über welche die Gleitführung des Schneidwerkzeugs auf der Führungsstange erfolgt. Beispielsweise kann die Länge im Bereich von 5 cm liegen. Dadurch kann eine stabile Führung des Schneidwerkzeugs erreicht werden.

Vorzugsweise weist das Instrument ein Gegenhaltestück auf, welches mit einem vorderen Ende der Führungsstange verbindbar und von diesem abnehmbar ist, wobei das Gegenhaltestück einen den Verschiebeweg des Schneidwerkzeugs auf der Führungsstange begrenzenden Anschlag für die Schneide aufweist. Nach dem Hindurchführen der Führungsstange durch den Fistelgang kann das Gegenhaltestück vom medizinischen Personal mittels eines medizinischen Bestecks mit der Führungsstange verbunden werden. Zur Verbindung eignen sich z.B. Schnappverbindungen oder andere im Stand der Technik bekannte Verbindungselemente. Besonders bevorzugt ist eine Schraubverbindung.

Durch das Gegenhaltestück wird das zu entfernende Körpergewebe an der der Schneide gegenüberliegenden Seite abgestützt, wobei der begrenzende Anschlag für die Schneide insbesondere einen sauberen Endschnitt ermöglicht, d.h. ein vollständiges Abtrennen der Fistelwand bzw. des zu entfernenden Körpergewebes. Vorzugsweise ist der Durchmesser des begrenzenden Anschlags größer als der Kreisdurchmesser des Kreises auf welchem die Schneidkante der Schneide liegt, um ein unbeabsichtigtes Hineinziehen des Gegenhaltestücks in den Fistelgang zu verhindern.

Vorteilhafterweise weist das Instrument zum Einführen der Führungsstange in den Fistelgang eine stabförmige und biegbare Einführhilfe auf, welche mit einem vorderen Ende der Führungsstange verbindbar und von diesem abnehmbar ist. Die Einführhilfe könnte auch als Einführsonde bezeichnet werden und ist günstigerweise einfach von Hand plastisch verformbar. Dadurch können insbesondere gekrümmte Fistelgänge sondiert werden, wodurch die Führungsstange in einen solchen gekrümmten Fistelgang unter Geraderichtung des Fistelgangs eingeführt werden kann. Der Durchmesser der Einführhilfe ist günstigerweise kleiner als der Durchmesser des Fistelgangs.

Die Führungsstange weist also günstigerweise eine größere Biegesteifigkeit als die Einführhilfe auf, sodass die Einführhilfe leichter, insbesondere wesentlich leichter, biegbar ist als die Führungsstange. Die Führungsstange wird bei der Benutzung des Instruments vorzugsweise nicht verformt. Dies ermöglicht die Aufrechterhaltung der Koaxialität der Schneide des Schneidwerkzeugs bezüglich der Führungsstange während des Schneidvorgangs. Der Außendurchmesser der Führungsstange beträgt günstigerweise mehr als 3 mm. Damit kann eine ausreichende Stabilität gegen Durchbiegungen der Führungsstange zur konzentrischen Führung der Schneide erreicht werden.

Vorteilhaft ist es, wenn die Führungsstange einen Haltegriff zur Erleichterung des Haltens der Führungsstange durch den Bediener aufweist, welcher, in einem Querschnitt orthogonal zur Längsmittelachse der Führungsstange gesehen, eine von der Kreisform abweichende Außenkontur aufweist. Dadurch kann beispielsweise die Verdrehung der Führungsstange, z.B. zum Aufschrauben des Gegenhaltestücks oder der Einführhilfe, erleichtert werden.

Die Führungsstange weist vorzugsweise eine Länge von zumindest 6 cm, besonders bevorzugt von zumindest 15 cm auf.

Das Schneidwerkzeug weist günstigerweise einen Bediengriff zur Erleichterung der Bedienung des Schneidwerkzeugs auf, welcher, in einem Querschnitt orthogonal zur Längsachse des Schneidwerkzeugs gesehen, eine von der Kreisform abweichende Außenkontur aufweist. Mit dem Bediengriff kann das Schneidwerkzeug relativ zur Führungsstange verdreht werden, um die Schneidebewegung auszuführen. Auch die Bewegung des Schneidwerkzeugs in Richtung der Längsmittelachse der Führungsstange kann dadurch erleichtert werden.

Vorteilhafterweise beträgt die in Richtung der Längsmittenachse des Schneidwerkzeugs gemessene Länge des Schneidabschnitts des Schneidwerkzeugs, und damit die Länge des ausgebildeten Aufnahmehohlraums, wenn das Schneidwerkzeug auf die Führungsstange aufgeschoben ist, zumindest 2 cm, vorzugsweise zumindest 4 cm, besonders bevorzugt zumindest 6 cm. Die Länge des Schneidabschnitts und damit die Länge des im auf die Führungsstange aufgeschobenen Zustand des Schneidwerkzeugs ausgebildeten Aufnahmehohlraums entspricht günstigerweise zumindest der Länge der zu entfernenden Fistelwand des Fistelgangs.

Vorzugsweise beträgt die Differenz zwischen dem Kreisdurchmesser des Kreises auf welchem die Schneidekante der Schneide, in einer axialen Ansicht auf das Schneidwerkzeug, liegt und dem Außendurchmesser der Führungsstange zumindest 1 mm, besonders bevorzugt zumindest 2 mm. Damit kann bei an der Führungsstange anliegendem Fistelgang ein hülsenförmiger Gewebebereich der entsprechenden Stärke abgetragen werden.

Weitere Merkmale und Einzelheiten der Erfindung werden anhand des in den Figuren gezeigten Ausführungsbeispiels eines Instruments zum Entfernen von Körpergewebe aus einem Fistelgang und anhand beispielhafter schematischer Darstellungen des Abtrennens von Körpergewebe im Bereich einer Analfistel erläutert. Es zeigen:
Fig. 1 eine isometrische Ansicht eines erfindungsgemäßen Instruments im Auslieferungszustand;
Fig. 2 das Instrument gemäß Fig. 1 mit vom Schneidwerkzeug abgenommener Schutzkappe;
Fig. 3 eine Seitenansicht des Instruments gemäß Fig. 2 ohne Schutzkappe;
Fig. 4 eine Detailansicht der mit der Führungsstange verbundenen Einführhilfe gemäß Fig. 3;
Fig. 5 die von der Führungsstange abgenommene Einführhilfe;
Fig. 6 eine Detailansicht des auf die Führungsstange aufgeschraubten Gegenhaltestücks;
Fig. 7 einen Längsmittelschnitt des Schneidwerkzeugs (Schnittebene parallel zur Zeichnungsebene in Fig. 3);
Fig. 8 eine isometrische Ansicht des auf die Führungsstange aufgeschobenen Schneidwerkzeugs, inklusive Gegenhaltestück;
Fig. 9 eine Seitenansicht auf einen vorderen Abschnitt des Instruments gemäß Fig. 8;
Fig. 10 einen Längsmittelschnitt durch den in Fig. 9 dargestellten Bereich des Instruments;
Fig. 11 den Längsmittelschnitt analog zu Fig. 10 mit bis zum Anschlag vorgeschobenem Schneidwerkzeug;
Fig. 12 den Schnitt A-A gemäß Fig. 9;
Fig. 13 eine schematische Schnittdarstellung einer Fistel im Bereich des Anus;
Fig. 14 bis 16 schematische Darstellungen verschiedener Schritte für das Abtrennen von Körpergewebe im Bereich des Fistelgangs gemäß Fig. 13, und
Fig. 17 das Detail B gemäß Fig. 16 in vergrößerter Darstellung.

Das Instrument zum Entfernen von Körpergewebe aus einem Fistelgang 1 weist ein rohrförmiges Schneidwerkzeug 10 auf. Die Form des Schneidwerkzeugs 10 könnte auch als hülsenförmig bezeichnet werden. Das Schneidwerkzeug 10 ist im Ausführungsbeispiel von einem ersten Rohrstück 18 und einem zweiten Rohrstück 19 gebildet, welche starr miteinander verbunden sind, vorzugsweise stoffschlüssig, besonders bevorzugt mittels Klebstoff 21, beispielsweise einem an sich bekannten Zweikomponenten-Klebstoff. Die Verbindung könnte beispielsweise auch durch Verpressung erfolgen. Das Schneidwerkzeug 10 besteht somit im Ausführungsbeispiel zumindest im Wesentlichen (also abgesehen vom Klebstoff 21) aus Metall. Dies ist aber nur eine mögliche Ausgestaltung eines erfindungsgemäßen Schneidwerkzeugs 10. Es könnte beispielsweise auch einstückig aus einem einzigen Rohling, insbesondere aus Metall, gefertigt sein. In anderen Ausgestaltungen könnte das zweite Rohrstück 19 beispielsweise auch aus Kunststoff und das erste Rohrstück 18 aus Metall oder Keramik gefertigt sein.

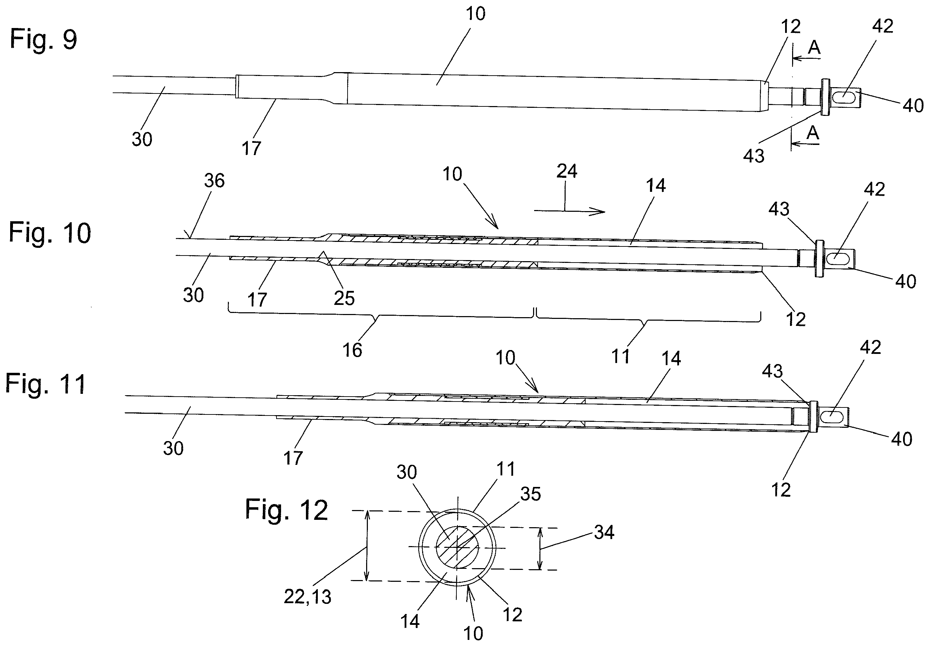

Das Schneidwerkzeug 10 weist einen, bezogen auf eine Schneidrichtung 24 des Schneidwerkzeugs 10, vorderen Schneidabschnitt 11 auf, an dem an einem vorderen Ende eine Schneide 12 ausgebildet ist, indem sich die Wand des Schneidwerkzeugs 10 am vorderen Ende des Schneidabschnitts 11 zu einer Schneidkante verjüngt. Die Schneidkante liegt in einer axialen Ansicht des Schneidwerkzeugs 10, d.h. orthogonal zur Längsmittelachse 23 des Schneidwerkzeugs 10 gesehen, auf einem Kreis, vgl. Fig. 12. Im Ausführungsbeispiel liegt die Schneidkante der Schneide 12 über ihren gesamten Umfang in einer Ebene, könnte aber beispielsweise auch einen Wellenschliff aufweisen.

Der Kreisdurchmesser 22 des Kreises auf welchem die Schneidkante der Schneide 12 liegt, beträgt weniger als 1 cm, im Ausführungsbeispiel beträgt der Kreisdurchmesser 22 0,6 cm. Der Kreisdurchmesser 22 kann je nach Querschnitt des Fistelgangs 1 bzw. der Stärke der Fistelwand durch Auswahl eines entsprechenden Schneidwerkzeugs 10 gewählt werden.

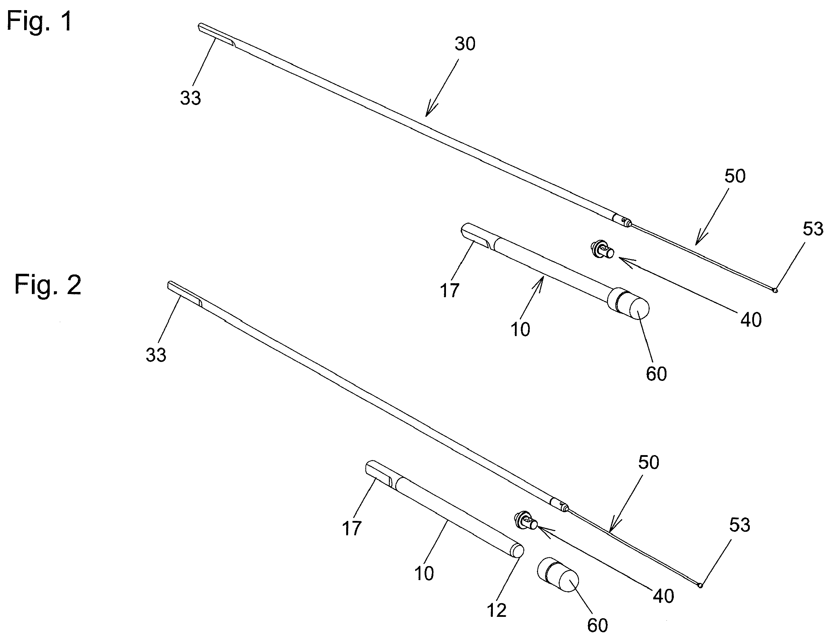

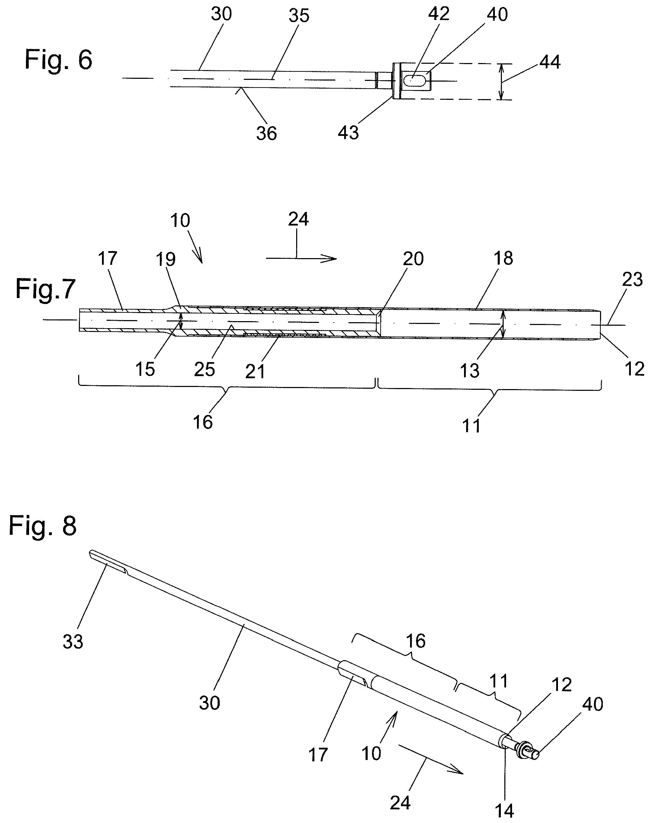

Die Schneide 12 weist im Ausführungsbeispiel eine kegelstumpfmantelförmige Außenseite auf, vgl. Fig. 7 und 8, könnte aber beispielsweise auch innen oder beidseitig abgeschrägt ausgeführt sein. Im Auslieferungszustand schützt die Schutzkappe 60 des Instruments die Schneide 12 vor Verschmutzung oder Beschädigung, vgl. Fig. 1.

Das Schneidwerkzeug 10 weist, bezogen auf die Schneidrichtung 24, einen hinteren Führungsabschnitt 16 zur verschiebbaren Führung des Schneidwerkzeugs 10 auf der noch näher zu erläuternden Führungsstange 30 auf. Über die gesamte axiale Erstreckung des Führungsabschnitts 16 ist der Innendurchmesser 15 des Führungsabschnitts 16 kleiner als der Innendurchmesser 13 des Schneidabschnitts 11 über die gesamte axiale Erstreckung des Schneidabschnitts 11, vgl. Fig. 7. In einem Übergangsbereich zwischen dem Schneidabschnitt 11 und dem Führungsabschnitt 16 ist günstigerweise eine Ansenkung (=Fase) 20 vorgesehen, welche das Aufschieben des Schneidwerkzeugs 10 auf die Führungsstange 30 erleichtert. Eine solche Ansenkung kann auch entfallen.

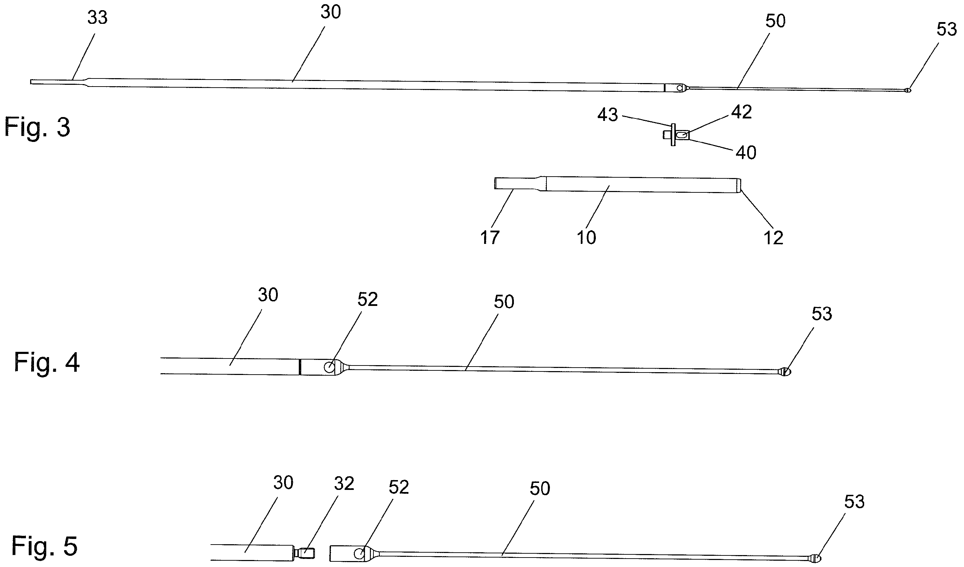

Um die Handhabe des Schneidwerkzeugs 10 für den Bediener zu erleichtern, weist das Schneidwerkzeug 10 an der Außenseite des Führungsabschnitts 16 günstigerweise einen Bediengriff 17 zur Erleichterung der Bedienung des Schneidwerkzeugs 10 auf. In einem Querschnitt orthogonal zur Längsmittelachse 23 des Schneidwerkzeugs 10 gesehen, weist der Bediengriff 17 eine von der Kreisform abweichende Außenkontur auf. Im Ausführungsbeispiel ist der Bediengriff 17 durch zwei gegenüberliegende Flachstellen ausgebildet, vgl. Fig. 3.

Die Führungsstange 30 ist längserstreckt und gerade. Sie weist günstigerweise über den Großteil ihrer Längserstreckung einen kreisförmigen Querschnitt auf, vgl. Fig. 3 und Fig. 12. Die Länge der Führungsstange 30 beträgt im Ausführungsbeispiel 25 cm.

Das Schneidwerkzeug 10 kann auf die Führungsstange 30 aufgeschoben werden, wobei der Führungsabschnitt 16 des Schneidwerkzeugs 10 mit seiner inneren Oberfläche 25 auf der äußeren Oberfläche 36 der Führungsstange 30 verschiebbar geführt ist. Die innere Oberfläche 25 des hinteren Führungsabschnitts 16 wirkt also zur Ausbildung einer Gleitführung des Schneidwerkzeugs 10 auf der Führungsstange 30 mit der äußeren Oberfläche 36 der Führungsstange 30 zusammen. Die innere Oberfläche 25 des Führungsabschnitts 16 ist vorzugsweise zylindermantelförmig ausgebildet.

Im auf die Führungsstange 30 aufgeschobenen Zustand des Schneidwerkzeugs 10 liegen das Schneidwerkzeug 10 und die Führungsstange 30 koaxial, d.h. die Längsmittelachse 23 des Schneidwerkzeugs 10 und die Längsmittelachse 35 der Führungsstange 30 liegen auf einer gemeinsamen Geraden. Auch der Kreis, auf welchem die Schneidkante der Schneide 12 liegt, liegt bei auf die Führungsstange 30 aufgeschobenem Zustand zur Führungsstange 30 koaxial, vgl. Fig. 12.

Die Führungsstange 30 besteht im Ausführungsbeispiel aus Metall. Sie wird bei der Benutzung quasi nicht verformt. Dadurch kann die Koaxialität der Schneide 12 in Bezug auf die Führungsstange 30 während des Schneidvorgangs gewährleistet werden. Damit kann ein gleichförmiges Abtrennen der Fistelwand über die gesamte Längserstreckung des Fistelgangs 1 erreicht werden, wie weiter unten genauer erläutert.

Der Innendurchmesser 15 des Führungsabschnitts 16 und der Außendurchmesser 34 der Führungsstange 30 bilden zusammen eine Spielpassung, um eine Gleitführung des Schneidwerkzeugs 10 auf der Führungsstange 30 auszubilden. Die Differenz des Innendurchmessers 15 des Führungsabschnitts 16 des Schneidwerkzeugs 10 und des Außendurchmessers 34 der Führungsstange 30 beträgt günstigerweise weniger als 0,2 mm (zumindest über den Großteil der Längserstreckung der Führungsstange 30, über welchen diese günstigerweise kreiszylindrisch ausgebildet ist).

Am hinteren Ende der Führungsstange 30 ist vorzugsweise ein Haltegriff 33 zur Erleichterung des Haltens der Führungsstange 30 durch den Operateur ausgebildet. In einem Querschnitt orthogonal zur Längsmittelachse 35 der Führungsstange 30 gesehen, weist die Führungsstange 30 im Bereich des Haltegriffs 33 eine von der Kreisform abweichende Außenkontur auf. Im Ausführungsbeispiel ist der Haltegriff 33 durch zwei gegenüberliegende Flachstellen an der Führungsstange 30 ausgebildet, vgl. Fig. 3.

Sowohl der Haltegriff 33 der Führungsstange 30, als auch der Bediengriff 17 des Schneidwerkzeugs 10 könnten in anderen Ausgestaltungen gemäß der Erfindung eine andere als die durch die Flachstellen des Ausführungsbeispiels ausgebildete Außenkontur, z.B. eine sternförmige Außenkontur, aufweisen.

Der Innendurchmesser 13 des Schneidabschnitts 11 des Schneidwerkzeugs 10 ist über die gesamte axiale Erstreckung des Schneidabschnitts 11 größer als der Außendurchmesser 34 der Führungsstange 30. Bei auf die Führungsstange 30 aufgeschobenem Schneidwerkzeug 10 wird dadurch ein Aufnahmehohlraum 14 für die Aufnahme von abgetrenntem Körpergewebe 5 ausgebildet. Dieser Aufnahmehohlraum 14 wird also nach innen von der äußeren Oberfläche 36 der Führungsstange 30 und nach außen von der inneren Oberfläche 25 des Schneidabschnitts 11 des Schneidwerkzeugs 10 begrenzt. Der Aufnahmehohlraum 14 ist also hülsenförmig oder rohrförmig und nach vorne (bezogen auf die Schneidrichtung 24) offen, vgl. Fig. 10 und 12. Der Aufnahmehohlraum 14 schließt direkt an die Schneidkante der Schneide 12 an. Der Kreisdurchmesser 22 des Kreises auf welchem die Schneidkante der Schneide 12 liegt entspricht im Ausführungsbeispiel dem Innendurchmesser 13 des Schneidabschnitts 11 des Schneidwerkzeugs 10.

Die Differenz zwischen dem Innendurchmesser 13 des Schneidabschnitts 11 und dem Außendurchmesser 34 der Führungsstange 30 beträgt vorzugsweise zumindest 1 mm, besonders bevorzugt zumindest 2 mm.

Der Außendurchmesser des Schneidwerkzeugs 10 ist über den Schneidabschnitt 11 konstant und über den Führungsabschnitt 16 zumindest nicht größer als im Schneidabschnitt 11.

Das Instrument weist im Weiteren eine stabförmige und biegbare Einführhilfe 50 auf, welche mit einem vorderen Ende der Führungsstange 30 verbindbar und von diesem abnehmbar ist. Zur Verbindung mit der Führungsstange 30 weist die Einführhilfe 50 im Ausführungsbeispiel eine Gewindebohrung auf (nicht sichtbar in den Figuren).

Am vorderen Ende der Führungsstange 30 ist ein korrespondierender Gewindezapfen 32 ausgebildet, vgl. Fig. 5. Am vorderen Ende der Einführhilfe 50 weist diese einen abgerundeten Kopf 53 auf, welcher das Einführen der Einführhilfe 50 in den Fistelgang 1 erleichtert. Beim Einführen in den Fistelgang 1 kann die Einführhilfe 50 dem Verlauf des Fistelgangs 1 durch ihre Biegsamkeit folgen, bzw. kann beim Einführen der Einführhilfe 50 in den Fistelgang 1 auch bereits ein gewisses Geraderichten des Fistelgangs 1 stattfinden.

Zur Erleichterung des Aufschraubens bzw. des Abschraubens der Einführhilfe 50 vom vorderen Ende der Führungsstange 30 kann die Einführhilfe 50 eine Aussparung 52 aufweisen, welche die Manipulation mittels eines medizinischen Bestecks erleichtert. Im Auslieferungszustand ist die Einführhilfe 50 günstigerweise bereits mit der Führungsstange 30 verbunden, um einen zusätzlichen Arbeitsgang zum Aufschrauben der Einführhilfe 50 zu vermeiden, vgl. Fig. 1.

Das Instrument weist im Weiteren ein Gegenhaltestück 40 auf, welches mit der Führungsstange 30 verbindbar und von dieser abnehmbar ist, vgl. Fig. 6. Analog zur Einführhilfe 50 weist das Gegenhaltestück 40 eine (in den Figuren nicht sichtbare) Gewindebohrung auf, welche mit dem Gewindezapfen 32 am vorderen Ende der Führungsstange 30 durch Aufschrauben verbindbar und von diesem wieder abnehmbar ist.

Das Gegenhaltestück 40 weist einen Anschlag 43 auf, welcher den Verschiebeweg des Schneidwerkzeugs 10 auf der Führungsstange 30 in die Schneidrichtung 24 begrenzt. Im weitestmöglich auf der Führungsstange 30 in die Schneidrichtung 24 verschobenen Zustand des Schneidwerkzeugs 10 stößt die Schneide 12 am Anschlag 43 an, vgl. Fig. 11.

Das Gegenhaltestück 40 kann eine Aussparung 42 aufweisen, um das Aufschrauben des Gegenhaltestücks 40 im Körperinneren während des Eingriffs zu erleichtern. Der Operateur kann das Gegenhaltestück 40 z.B. mittels einer Pinzette festhalten und die Führungsstange 30 drehen, um so die Verbindung mit der Führungsstange 30 herzustellen.

Vorzugsweise schlägt die Schneidkante der Schneide 12 beim Verschieben des Schneidwerkzeugs 10 umfänglich am Anschlag 43 an, um ein sauberes Abtrennen am Ende des Verschiebeweges des Schneidwerkzeugs 10 auf der Führungsstange 30 zu ermöglichen, vgl. Fig. 11. Im Ausführungsbeispiel ist der Durchmesser 44 nur geringfügig größer wie der Kreisdurchmesser 22, könnte in anderen Ausgestaltungen der Erfindung aber deutlich größer sein.

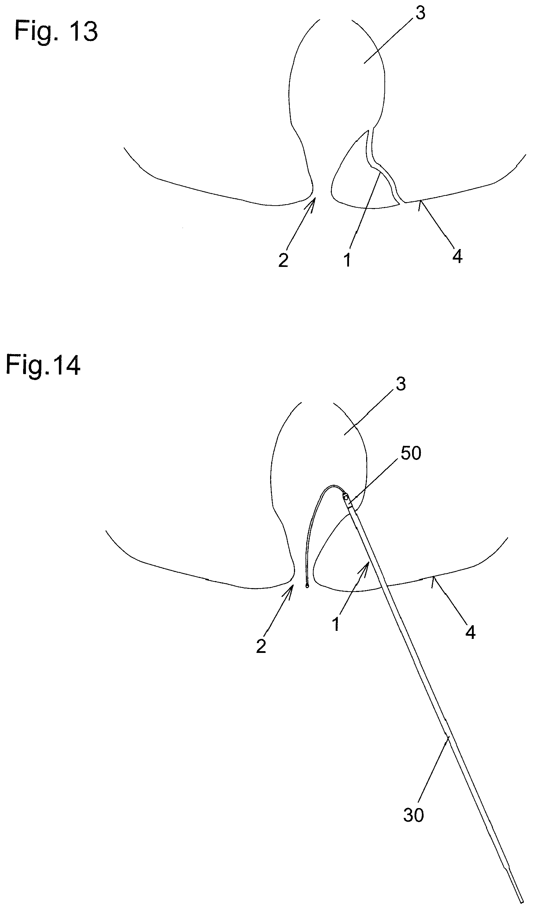

Im Folgenden wird anhand der Fig. 13 bis 17 der Vorgang des Entfernens von Körpergewebe aus einem Fistelgang 1 im Bereich des Anus erläutert. Der Fistelgang 1 bildet einen Kanal zwischen dem Rektum 3 und der Körperoberfläche 4. Der Verlauf des Fistelgangs 1 ist nicht gerade, wie dies in der Praxis im Allgemeinen anzutreffen ist, vgl. Fig. 13. Im dargestellten Beispiel beträgt die Länge des Fistelgangs etwa 5 cm. Die den Fistelgang 1 begrenzende, in den Figuren nicht gesondert eingezeichnete, Fistelwand ist eine epitheliale Auskleidung, d.h. sie wird von einer Art dünnen Haut gebildet, deren Stärke üblicherweise im Bereich von 0,5 mm bis 1 mm liegt.

Zunächst wird die Führungsstange 30 mittels der Einführhilfe 50 in den Fistelgang 1 eingeführt. Der Fistelgang 1 wird durch die eingeführte Führungsstange 30 gerade gerichtet, vgl. Fig. 14. Es ist dabei zu beachten, dass der Außendurchmesser 34 der Führungsstange 30 günstigerweise größer gewählt wird, als der Durchmesser des Fistelgangs 1. Dadurch liegt die Fistelwand des Fistelgangs 1 im vollständig eingeführten Zustand der Führungsstange 30 vollständig an der Führungsstange 30 an.

In einem nächsten Schritt wird die Einführhilfe 50 von der Führungsstange 30 abgenommen. Hierzu wird die Einführhilfe 50, z.B. mit einem medizinischen Instrument, z.B. einer Pinzette, verdrehfest gehalten, während die Führungsstange 30, z.B. mittels des Haltegriffs 33, gegenüber dem Fistelgang 1 und der Einführhilfe 50 verdreht wird. Nach dem Abschrauben und Entfernen der Einführhilfe 50 wird das Gegenhaltestück 40 mit dem vorderen Ende der Führungsstange 30, welches sich im Inneren des Körpers befindet, verbunden. Auch hierzu kann die Führungsstange 30 günstigerweise mittels des Haltegriffs 33 gegenüber dem festgehalten Gegenhaltestück 40 verdreht werden. Das Gegenhaltestück 40 bildet im mit der Führungsstange 30 verbundenen Zustand günstigerweise auch eine Art Anker, um zu verhindern, dass das vordere Ende der Führungsstange 30 während des Schneidvorgangs in den Fistelgang 1 rutscht.

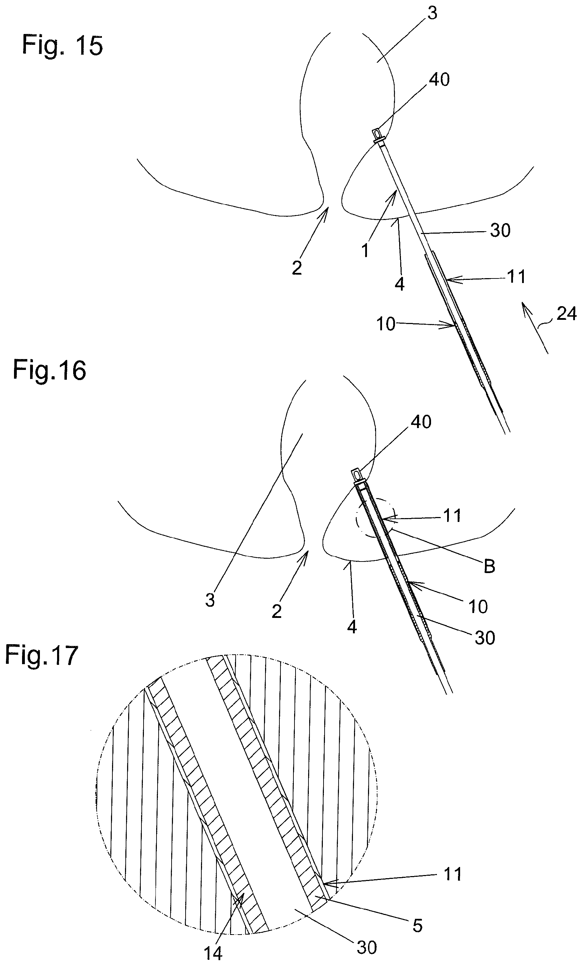

Im darauf folgenden Arbeitsschritt wird das Schneidwerkzeug 10 auf die Führungsstange 30 aufgeschoben, vgl. Fig. 15. Während des folgenden Schneidvorgangs wird das Schneidwerkzeug 10 günstigerweise zusätzlich zur Bewegung in Schneidrichtung 24 gegenüber der Führungsstange 30 verdreht, um einen sauberen Schnitt zu ermöglichen. Das während des Schneidvorgangs bereits abgetrennte Körpergewebe 5 wird nach und nach im Aufnahmehohlraum 14 zwischen dem Schneidabschnitt 11 des Schneidwerkzeugs 10 und der Führungsstange 30 aufgenommen, vgl. Fig. 16 und 17.

Am Ende des Verschiebeweges des Schneidwerkzeugs 10 steht die Schneide 12 des Schneidabschnitts 11 am Anschlag 43 des Gegenhaltstücks 40 an. Im Aufnahmehohlraum 14 befindet sich das vom umliegenden Gewebe des Körpers vollständig abgetrennte Körpergewebe 5, welches die epitheliale Auskleidung, d.h. die Fistelwand des Fistelgangs 1, umfasst.

In einem letzten, nicht gesondert dargestellten Arbeitsschritt, wird die Führungsstange 30 mit dem Schneidwerkzeug 10 und dem abgetrennten Körpergewebe 5 aus dem Körper herausgeführt. Das Gegenhaltestück 40 kann vorher entfernt werden, insbesondere wenn ein Gegenhaltestück 40 eingesetzt wird, dessen Durchmesser 44 deutlich größer als der Außendurchmesser des Schneidwerkzeugs 10 ist. Alternativ könnte, insbesondere wenn der Durchmesser des Gegenhaltestücks 40 annähernd dem Kreisdurchmesser 22 entspricht, das Gegenhaltestück 40 gemeinsam mit der Führungsstange 30 und dem Schneidwerkzeug 10 aus dem Körper des Patienten herausgezogen werden.

Nach Entfernen der Fistelwand liegt frisches, durchblutetes Körpergewebe im Bereich des nun in seinem Querschnitt erweiterten Fistelgangs 1 vor. Das durchblutete Gewebe ermöglicht ein Zusammenwachsen und Ausheilen der Fistel nach dem Eingriff.

Im gezeigten Ausführungsbeispiel beträgt die Differenz zwischen dem Kreisdurchmesser 22 des Kreises auf welchem die Schneidkante der Schneide 12 liegt und dem Außendurchmesser 34 der Führungsstange 30 2 mm. Dadurch kann die Fistelwand (welche z.B. eine Stärke von 0,5 mm aufweist) und an die Fistelwand angrenzendes Körpergewebe (z.B. mit einer Stärke von 0,5 mm) zur Gänze über die gesamte Längserstreckung des Fistelgangs 1 entfernt werden. In anderen Worten kann hülsenförmiges Körpergewebe, im Ausführungsbeispiel mit einer Wandstärke von 1 mm, mit dem dargestellten Instrument entfernt werden.

Je nach Länge und Durchmesser des zu behandelnden Fistelgangs und der Stärke der Fistelwand können Instrumente mit entsprechenden Abmessungen vorgesehen sein.

**Legende zu den Hinweisziffern:**

| | | | |
|---|---|---|---|
| 1 | Fistelgang | 30 | Führungsstange |
| 2 | Anus | 31 | Längsmittelachse |
| 3 | Rektum | 32 | Gewindezapfen |
| 4 | Körperoberfläche | 33 | Haltegriff |
| 5 | abgetrenntes Körpergewebe | 34 | Außendurchmesser |
| | | 35 | Längsmittelachse |
| 10 | Schneidwerkzeug | 36 | äußere Oberfläche |
| 11 | Schneidabschnitt | | |
| 12 | Schneide | 40 | Gegenhaltestück |
| 13 | Innendurchmesser | 42 | Aussparung |
| 14 | Aufnahmehohlraum | 43 | Anschlag |
| 15 | Innendurchmesser | 44 | Durchmesser |
| 16 | Führungsabschnitt | | |
| 17 | Bediengriff | 50 | Einführhilfe |
| 18 | erstes Rohrstück | 52 | Aussparung |
| 19 | zweites Rohrstück | 53 | Kopf |
| 20 | Ansenkung | | |
| 21 | Klebstoff | 60 | Schutzkappe |
| 22 | Kreisdurchmesser | | |
| 23 | Längsmittelachse | | |
| 24 | Schneidrichtung | | |
| 25 | innere Oberfläche | | |

## Patentansprüche

1. Instrument zum Entfernen von Körpergewebe aus einem Fistelgang (1) mit einem eine Schneide (12) aufweisenden Schneidwerkzeug (10), **dadurch gekennzeichnet, dass** das Instrument im weiteren eine Führungsstange (30) zum Durchführen durch den Fistelgang (1) aufweist und dass das Schneidwerkzeug (10) rohrförmig ausgebildet ist und auf die Führungsstange (30) aufschiebbar ist und einen hinteren Führungsabschnitt (16) zur verschiebbaren Führung des Schneidwerkzeugs (10) auf der Führungsstange (30) und einen vorderen Schneidabschnitt (11) aufweist, an dem an einem vorderen Ende die Schneide (12) ausgebildet ist und dessen Innendurchmesser (15) zur Ausbildung eines Aufnahmehohlraums (14) für die Aufnahme von abgetrenntem Körpergewebe (5) größer als der Außendurchmesser (34) der Führungsstange (30) ist.

2. Instrument nach Anspruch 1, **dadurch gekennzeichnet, dass** die Schneidkante der Schneide (12) in einer axialen Ansicht des Schneidwerkzeugs (10) auf einem Kreis liegt, welcher beim auf die Führungsstange (30) aufgeschobenen Schneidwerkzeug (10) konzentrisch zur Führungsstange (30) liegt.

3. Instrument nach Anspruch 2, **dadurch gekennzeichnet, dass** der Kreis einen Kreisdurchmesser (22) von höchstens 1 cm aufweist.

4. Instrument nach einem der Ansprüche 1 bis 3, **dadurch gekennzeichnet, dass** das Instrument ein Gegenhaltestück (40) aufweist, welches mit einem vorderen Ende der Führungsstange (30) verbindbar und von diesem abnehmbar ist, wobei das Gegenhaltestück (40) einen den Verschiebeweg des Schneidwerkzeugs (10) auf der Führungsstange (30) begrenzenden Anschlag (43) für die Schneide (12) aufweist.

5. Instrument nach einem der Ansprüche 1 bis 4, **dadurch gekennzeichnet, dass** der Führungsabschnitt (16) bezogen auf die Richtung der Längsmittelachse (23) des Schneidwerkzeugs (10) eine Länge von zumindest 1 cm, vorzugsweise von zumindest 3 cm, aufweist.

6. Instrument nach einem der Ansprüche 1 bis 5, **dadurch gekennzeichnet, dass** das Instrument zum Einführen der Führungsstange (30) in den Fistelgang (1) eine stabförmige und biegbare Einführhilfe (50) aufweist, welche mit einem vorderen Ende der Führungsstange (30) verbindbar und von diesem abnehmbar ist.

7. Instrument nach einem der Ansprüche 1 bis 6, **dadurch gekennzeichnet, dass** die Führungsstange (30) einen Haltegriff (33) zur Erleichterung des Haltens der Führungsstange (30) durch den Bediener aufweist, welcher, in einem Querschnitt orthogonal zur Längsmittelachse (35) der Führungsstange (30) gesehen, eine von der Kreisform abweichende Außenkontur aufweist und/oder dass das Schneidwerkzeug (10) einen Bediengriff (17) zur Erleichterung der Bedienung des Schneidwerkzeugs (10) aufweist, welcher, in einem Querschnitt orthogonal zur Längsmittelachse (23) des Schneidwerkzeugs (10) gesehen, eine von der Kreisform abweichende Außenkontur aufweist.

8. Instrument nach einem der Ansprüche 1 bis 7, **dadurch gekennzeichnet, dass** der im auf die Führungsstange (30) aufgeschobenen Zustand des Schneidwerkzeugs (10) ausgebildete Aufnahmehohlraum (14) bezogen auf die Richtung der Längsmittelachse (23) des Schneidwerkzeugs (10) eine Länge von zumindest 2 cm, vorzugsweise von zumindest 4 cm, aufweist.

9. Instrument nach einem der Ansprüche 1 bis 8, **dadurch gekennzeichnet, dass** die Führungsstange (30) eine Länge von zumindest 6 cm, vorzugsweise von zumindest 15 cm, aufweist.

10. Instrument nach einem der Ansprüche 1 bis 9, **dadurch gekennzeichnet, dass** die Differenz zwischen dem Innendurchmesser (13) des Schneidabschnitts (11) und dem Außendurchmesser (34) der Führungsstange (30) zumindest 1 mm, vorzugsweise zumindest 2 mm, beträgt.
